# EUROPÄISCHE PATENTSCHRIFT

(11) **EP 1 702 595 B1**
(45) Veröffentlichungstag und Bekanntmachung des Hinweises auf die Patenterteilung: **01.12.2010**
(21) Anmeldenummer: 05006033.4
(22) Anmeldetag: 18.03.2005
(51) Int. Cl.: A61F 9/01

(54) **Vorrichtung zur ophthalmologischen Laserchirurgie**
Apparatus for ophthalmologic laser surgery
Dispositif pour la chirurgie ophthalmologique par laser

(43) Veröffentlichungstag der Anmeldung: 20.09.2006
(60) Teilanmeldung: 10010336.5
(73) Patentinhaber: WaveLight GmbH, 91058 Erlangen (DE)
(72) Erfinder: Donitzky, Christof, 90542 Eckental (DE); Klafke, Mario, 63814 Mainaschaff (DE)
(74) Vertreter: von Hellfeld, Axel

(56) Entgegenhaltungen:
- EP-A- 1 210 923
- WO-A1-2004//003468
- WO-A2-03/082162
- US-A- 6 099 522
- US-A1- 2002 169 441
- US-A1- 2003 144 650
- US-B1- 6 592 574

## Beschreibung

Die Erfindung betrifft eine Vorrichtung zur ophthalmologischen Laserchirurgie mit einem Laser zum Emittieren eines refraktiv wirkenden Laserstrahls, optischen Elementen zum Formen und Richten des Laserstrahls auf ein Auge, und einem Rechner zum Steuern des Lasers und/oder der optischen Elemente.

In der Ophthalmologie hat der Laser vielfältige Anwendungen gefunden, insbesondere in der refraktiven Chirurgie, also der therapeutischen Wirkung des Lasers auf das Auge zur Veränderung von dessen Brechungseigenschaften. Ein refraktiv wirkender Laserstrahl kann zum Beispiel die Brechungseigenschaften des Auges dadurch ändern, dass der Strahl von der Cornea Gewebe abträgt und so die Form der Cornea ändert. Ein bekanntes Beispiel hierfür ist das LASIK-Verfahren.

Der Stand der Technik kennt auch eine Vielzahl von Geräten und Einrichtungen (Sensoren), mit denen Eigenschaften eines Auges ermittelt werden können. Im Stand der Technik werden diese Sensoren eingesetzt, um vor Beginn des laserchirurgischen Eingriffs die erforderlichen Steuerdaten für die Steuerung der Laserstrahlung zu gewinnen, also insbesondere die Laserintensität und die Daten für die Steuerung des Laserstrahls in Zeit und Raum während des chirurgischen Eingriffs. So kennt der Stand der Technik zum Beispiel Mittel, mit denen bei einem zu behandelnden Auge die Gestalt der Vorderfläche der Cornea gemessen werden kann (Topographiemessgerät). Bekannt sind auch Mittel, mit denen bei einem zu behandelnden Auge die Wellenfrontaberration gemessen werden kann, zum Beispiel mit einem sogenannten Hartmann-Shack-Aberrometer oder auch gemäß dem Verfahren von Tscherning. Auch bekannt sind sogenannte Pachymeter, mit denen die Dicke (Stärke) der Hornhaut an verschiedenen Stellen derselben gemessen werden kann. Spektrometer werden im Stand der Technik eingesetzt, um Eigenschaften der Hornhaut zu ermitteln. Dabei kann Strahlung auf die Hornhaut gerichtet werden, die eine Emission, insbesondere Fluoreszenz, und/oder Reflexion von Strahlung von der Hornhaut oder anderen Augenteilen bewirkt und diese Strahlung kann mit Blick auf ihre Intensität und/oder Wellenlänge vermessen werden, um daraus auf Eigenschaften des Auges zu schließen.

Den nächsten Stand der Technik bilden US 6 099 522 und US 2003/0 144 650, in denen aufgrund der mit solchen Sensoren ermittelten Laser- und evtl. Augeneigenschaften dann die Programme generiert werden, mit denen ein Rechner einen Laserstrahl und die zugehörigen optischen Elemente zum Formen und Richten des Laserstrahls auf das Auge steuert, um ein gewünschtes Behandlungsergebnis zu erzielen. In diese Programme fließen dabei vielfältige Erfahrungswerte ein, die bezüglich der Wirkung der Laserstrahlung auf das Auge gewonnen worden sind. Allerdings können solche Erfahrungswerte nicht in jedem Einzelfall einer laserchirurgischen Operation das tatsächlich bei einem Patienten erzielte Ergebnis mit Sicherheit voraussagen.

Der Erfindung liegt die Aufgabe zugrunde, eine Vorrichtung zur ophthalmologischen Laserchirurgie der eingangs genannten Art so auszubilden, dass mit ihr in der Regel verbesserte Behandlungsergebnisse erzielt werden können.

Erfindungsgemäß weist die Vorrichtung hierfür einen Sensor auf, der eingerichtet ist, eine Eigenschaft des Laserstrahls während der Chirurgie zu ermitteln und ein dieser Eigenschaft entsprechendes Signal an den Rechner zu geben, der programmiert ist, um den Laser und/oder die optischen Elemente in Abhängigkeit von diesem Signal zu steuern, wie in Anspruch 1 definiert.

Gemäß der Erfindung kann es sich bei dem Sensor um eine oder mehrere der nachfolgenden Einrichtungen handeln: ein Pachymeter, ein Spektrometer, ein Aberrometer, eine Kamera, eine Einrichtung zur Messung einer Ablationstiefe, und zumindest eine Einrichtung, um während einer Operation eine Eigenschaft (einen Parameter) des Laserstrahles selbst zu messen, insbesondere dessen Energie und/oder dessen Qualität. Letzterer Lösungsgedanke berücksichtigt, dass sich Eigenschaften des Laserstrahls während der Operation ändern können, sodass es erforderlich werden kann, zur Erzielung eines optimalen Behandlungsergebnisses die laufende Steuerung des Lasers und/oder der den Laserstrahl formenden und führenden optischen Elemente in Abhängigkeit von der Änderung des Parameters einzustellen oder, im Extremfall, die Operation ganz zu unterbrechen.

Nach der Erfindung wird also während, d.h. im Verlaufe des laserchirurgischen Eingriffes nicht nur therapiert, sondern laufend eine Eigenschaft des Laserstrahls und des gerade behandelten Auges ermittelt und in Abhängigkeit von der ermittelten Eigenschaft der laufende chirurgische Eingriff dann gegenüber dem ursprünglichen (Ausgangs-) Bearbeitungsprogramm abgeändert, wenn die gerade ermittelte Eigenschaft dies sinnvoll erscheinen lässt. Es wird also die Erkenntnis berücksichtigt, dass der Verlauf einer laserchirurgischen Behandlung beim gerade behandelten Patienten anders sein kann, als ursprünglich bei Beginn der Behandlung angenommen wurde. Das ursprünglich bei Beginn der Behandlung zugrundegelegte Behandlungsprogramm basiert auf statistischen Erfahrungswerten, von denen ein individueller Patient mehr oder weniger abweichen kann. Die Erfindung ermöglicht also eine bessere Anpassung der durchgeführten Laserchirurgie an die Gegebenheiten des behandelten Patienten und damit in der Regel ein besseres Behandlungsergebnis. Anders ausgedrückt, ermöglicht die Erfindung eine Diagnose während der laserchirurgischen Therapie in der Art einer rückgekoppelten Regelung, bei der während des Verlaufes des therapeutischen Eingriffs Diagnosedaten laufend verrechnet werden, um daraus Steuerdaten für den anschließenden weiteren Verlauf der Therapie abzuleiten.

Zum Beispiel kann der erfindungsgemäße Rechner so programmiert sein, dass im ursprünglichen Behandlungsprogramm, also dem Programm, gemäß dem der laserchirurgische Eingriff begonnen wird, nach bestimmten zeitlichen Teilabschnitten der Gesamtbehandlung jeweils bestimmte Behandlungsziele aufgrund empirischer Daten (also mit anderen Patienten oder Testläufen gewonnener Daten) angenommen werden, zum Beispiel eine Ablationstiefe oder eine bestimmte Reststärke der Cornea an jeweils beobachteten Stellen. Nach der Erfindung wird dann nach den jeweiligen zeitlichen Teilabschnitten der laserchirurgischen Behandlung geprüft, ob die ursprünglich angenommene Ablationstiefe bzw. Reststärke der Cornea mit dem tatsächlich erreichten Zwischenergebnis der Behandlung übereinstimmt oder nicht. Ist die Ablationstiefe größer als ursprünglich angenommen, wird das nachfolgende laserchirurgische Programm dahingehend abgewandelt, dass die anschließende Ablation entsprechend geringer ist. Ist die Ablationstiefe geringer als angenommen, wird das Programm hingegen dahingehend abgeändert, dass bei der anschließenden Behandlung mehr Corneagewebe ablatiert (abgetragen) wird als ursprünglich vorgesehen. Analoges gilt für andere während des Verlaufs der laserchirurgischen Behandlung ermittelte und insbesondere gemessen Eigenschaften des behandelten Auges. Gemäß einer bevorzugten Ausgestaltung der Erfindung können die Zeitabschnitte, nach denen jeweils der vorstehend beschriebene Vergleich von Ist-Werten und Soll-Werten durchgeführt wird, mit sehr engem Raster über die gesamte Behandlung verteilt gewählt werden, d.h. es erfolgt nach einer Vielzahl von zeitlichen Zwischenintervallen der Behandlung der beschriebene Vergleich von Ist- und Soll-Werten.

Eine weitere Verbesserung des Behandlungsergebnis ist dadurch möglich, dass vorgelagert der vorstehend beschriebenen Berücksichtigung individueller Behandlungsergebnisse beim Patienten im Verlaufe des laserchirurgischen Eingriffs berücksichtigt wird, dass Menschen unterschiedlichen Typs (Rasse), empirisch gesehen, unterschiedlich auf die refraktive Laserchirurgie reagieren. So können hellhäutige Patienten und dunkelhäutige Patienten im statistischen Vergleich unterschiedliche Reaktionen auf den Laserstrahl zeigen und dies kann schon beim Erstellen des Behandlungsprogramms, das am Anfang der Behandlung zugrunde gelegt wird, berücksichtigt werden.

In analoger Weise können sich möglicherweise im Verlaufe der Operation ändernde Eigenschaften des Laserstrahls, wie zum Beispiel dessen Energie oder seine Qualität während der Behandlung nach einer Vielzahl von zeitlichen Behandlungsabschnitten überprüft und die Ist-Werte mit Soll-Werten in der beschriebenen Weise unter Anpassung des sich anschließenden Behandlungsverlaufes berücksichtigt werden. Erhöht sich zum Beispiel während der Behandlung die Energie des Lasers, dann wird das Programm dies dahingehend berücksichtigen, dass die anschließende Zahl der Laserpulse entsprechend verringert wird. Auch hier ist im Extremfall die Möglichkeit eines Abbruches der Behandlung gegeben, wenn eine Weiterführung der Behandlung zu undefinierten Risiken führen könnte.

Bevorzugt wird nach der Erfindung eine möglichst große Anzahl der vorstehend abgehandelten Augeneigenschaften und Laserstrahleigenschaften mit entsprechenden Sensoren während der Behandlung berücksichtigt. Nach der Erfindung können also einzelne der hier genannten Eigenschaften bzw. Laserstrahlungsparameter berücksichtigt werden oder bevorzugt mehrere oder besonders bevorzugt alle oder nahezu alle der genannten Eigenschaften bzw. Laserstrahlparameter.

Der Begriff "Sensor" im Sinne dieser Erfindung ist allgemein zu verstehen als eine Einrichtung, mit der die genannten Eigenschaften ermittelt werden können.

Bevorzugt wird die Erfindung in der PRK oder LASIK eingesetzt. Dann ist die Cornea Gegenstand der Diagnose des Sensors. Entsprechend der Anzahl der während der Behandlung berücksichtigten Eigenschaften der Cornea und der Laserstrahlung sind dann mehrere Sensoren für die jeweiligen Eigenschaften und Strahlungsparameter vorgesehen.

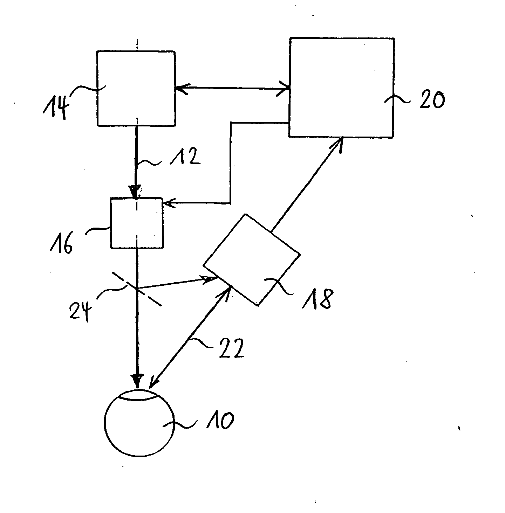

Nachfolgend werden Ausführungsbeispiele der Erfindung unter Bezugnahme auf die Zeichnung näher beschrieben.

Die einzige Figur zeigt schematisch eine Vorrichtung für die ophthalmologische Laserchirurgie.

Ein Auge 10 soll mit Laserstrahlung refraktiv behandelt werden. Zum Beispiel kann es sich bei der in der Figur gezeigten Vorrichtung um eine LASIK-Vorrichtung handeln. Nachfolgend wird die Erfindung unter nicht einschränkender Bezugnahme auf die LASIK-Technik beschrieben.

Ein Excimerlaser 14 emittiert einen gepulsten Laserstrahl 12, zum Beispiel mit einer Wellenlänge von 193 nm. An sich bekannte optische Elemente 16 formen und führen den Laserstrahl in Richtung auf das Auge 10.

Das Bezugszeichen 18 bezeichnet einen oder mehrere Sensoren, die angeordnet sind, um während der refraktiven Chirurgie Eigenschaften der Laserstrahlung und/oder der Cornea zu ermitteln. Das Bezugszeichen 22 deutet diese Ermittlung von Eigenschaften der Cornea symbolisch an. Zum Beispiel kann der Sensor 18 ein Pachymeter sein, welches in an sich bekannter Weise die Stärke der Hornhaut misst.

Zur Ermittlung von Eigenschaften der Laserstrahlung ist ein teildurchlässiger Spiegel 24 im Strahlengang angeordnet, um einen geringen Teil der Laserstrahlung aus dem Strahl heraus in einem Sensor 18 zu reflektieren. Bei einer solchen Messung ist der Sensor 18 zum Beispiel ausgelegt, um die Energie der Strahlung zu messen und/oder die Strahlungsqualität (Divergenz etc.). All diese Messungen und die Mittel dafür sind dem Fachmann als solches bekannt.

Die mit dem Sensor 18 gewonnenen Daten über die Eigenschaften der Laserstrahlung 12 und/oder der Cornea werden in Form eines elektrischen Signales in einen Rechner 20 eingegeben, der in an sich bekannter Weise den Laser 14 und/oder die optischen Elemente 16 ansteuert. Der Rechner 20 kann zunächst in herkömmlicher Weise für einen gegebenen Patienten programmiert sein, dessen Auge mit einem bestimmten Ziel hinsichtlich seiner Brechungseigenschaft gemäß dem LASIK-Verfahren neu geformt werden soll.

Über diesen Stand der Technik hinausgehend ist der Rechner 20 so programmiert, dass er nach regelmäßigen Zeitintervallen Ermittlungs- bzw. Messergebnisse vom Sensor 18 abfragt. Zum Beispiel kann die gesamte anfänglich vorgesehene Behandlungsdauer in n gleich lange Zeitintervalle aufgeteilt werden, nach denen der Rechner 20 vom Sensor 18 die Ermittlungs- bzw. Messdaten erhält. Die Zahl n kann dabei größer als 10, größer als 30, größer als 60 oder größer als 100 sein, je nach der Art der Daten, die der Sensor 18 gewinnt und dem Einfluss, den diese Daten auf den Verlauf der refraktiven Chirurgie haben.

Leistet der Sensor 18 eine berührungslose Pachymetrie, dann misst und speichert er die jeweilige momentane Corneastärke. Nach dem sogenannten LASIK-Schnitt und dem Hochklappen des Flap" wird wiederum die verbleibende Corneastärke gemessen und gespeichert. Im Verlauf der sukzessiven Ablation gemäß bekannten Steueralgorithmen misst der Sensor 18 permanent die jeweilige Stärke des Reststromas und vergleicht diese mit Soll-Werten der Stärke des Stromas, die gemäß dem Ablationsprogramm theoretisch erwartet wurden. Weicht bei diesem Vergleich der Ist-Wert der Stromastärke von deren Soll-Wert um mehr als eine vorgegebene Toleranz ab, berücksichtigt das Programm dies bei den anschließenden Arbeitsschritten. War die Ablation von Corneagewebe stärker als ursprünglich bei Beginn des Programms angenommen und im Programm vorausgesetzt, dann reduziert das Programm anschließend gegenüber dem Ausgangsprogramm die Ablation entsprechend. Umgekehrtes gilt im Falle, dass die während des Verlaufs der Behandlung erreichte Ablation geringer war als ursprünglich im Programm vorausgesetzt, sodass anschließend stärker ablatiert wird als ursprünglich geplant, d.h. die Zahl der Laserpulse bezogen auf die gerade betrachtete Flächeneinheit wird entsprechend erhöht.

Entsprechend können zusätzlich zur Zahl der Laserpulse auch die Laserenergie und/oder der Laserspot in an sich bekannter Weise verändert werden.

Der Sensor 18 kann auch in an sich bekannter Weise mittels eines Rechners eine Funktion aufbereiten, die den als einzelnen Punkten ermittelten Messwerten entspricht. Die so erzeugte Funktion und ihre jeweiligen örtlichen Steigungen (Gradienten) erlauben eine Feststellung zu dem Verlauf (Ergebnis) der laufenden Behandlung und diese Feststellung kann dann dazu dienen, den weiteren Verlauf der Behandlung entsprechend anzupassen.

Der Sensor 18 kann zusätzlich auch ein Spektrometer aufweisen, um berührungslos Eigenschaften der Cornea zu ermitteln, zum Beispiel den Feuchtegrad, der bekanntlich Einfluss auf die Ablationswirkung der Laserstrahlpulse hat. Ähnlich wie beim oben unter Bezugnahme auf das Pachymeter beschriebene Verfahren, wird das Spektrometer auch in den oben genannten Zeitintervallen, d.h. praktisch permanent, den Feuchtigkeitsgrad der Cornea überwachen und entsprechend dieser Überwachung werden Eigenschaften der Laserstrahlungspulse, zum Beispiel deren Energie, Abmessungen, Anzahl und Zeitfolge mehr oder weniger neu im Vergleich zum ursprünglich vorgesehenen Programm eingestellt. Dabei wird auch präoperativ der Feuchtigkeitsgrad festgestellt und davon ausgehend das anfängliche Programm für die refraktive Chirurgie in bekannter Weise erzeugt. Darüber hinausgehend wird dann auch während der Behandlung in der beschriebenen Weise nach jeweils geringen Zeitintervallen, also praktisch permanent, der Feuchtigkeitsgrad ermittelt und für das jeweils anschließende Programm berücksichtigt.

Der Sensor 18 kann zusätzlich auch eine Einzelpuls-Kamera aufweisen, die jeweils nach einem Laserpuls (oder einer Gruppe von Laserpulsen) einen Vergleich der Ist-Position des Laserstrahls auf der Cornea mit einer Soll-Position vornimmt. Es erfolgt also im Rechner 20 eine Bildverarbeitung. Weicht die Ist-Position um mehr als eine vorgegebene Toleranz von der Soll-Position des Laserpulses auf der Cornea ab, dann kann zum Beispiel ein Abschalten der Vorrichtung vorgesehen sein. Das Merkmal "Steuern des Lasers und/oder der optischen Elemente" beinhaltet also auch ein Abschalten des Lasers bzw. ein Unterbinden des Einfalls der Laserstrahlung auf die Cornea. Bei Einsatz einer Einzelpuls-Kamera, die das Ergebnis jedes Laserpulses auf der Cornea fotografiert, können auch die einzelnen Laserpulse, die am selben Ort auf die Cornea auftreffen, gezählt werden. Treffen mehr als eine vorgegebene Anzahl von Laserpulsen auf dieselbe Stelle der Cornea, dann zeigt dies ebenfalls einen Fehler des Systems an und infolge dessen kann das Programm ein Abschalten des Lasers für diesen Fall vorsehen.

Der Sensor 18 kann außer einem oder mehreren der vorstehend abgehandelten Messeinrichtungen (wobei der Begriff "messen" auch im allgemeinen Sinn als Ermitteln von Informationen zu verstehen ist) auch eine Einrichtung zur Bestimmung der jeweils momentan erreichten Ablationstiefe an verschiedenen Stellen der Cornea aufweisen. Auch hier kann ein Vergleich von Ist-Werten und Soll-Werten im oben beschriebenen Sinne analog durchgeführt werden und entsprechend das anschließende Laserprogramm daran angepasst werden.

Weiterhin kann der Sensor 18 eine optische Messeinrichtung aufweisen, um eine automatische Zentrierung der Ablationszone zu bewirken. Damit wird eine unbeabsichtigte "Wanderung" des Laserstrahls aus der Soll-Position erfasst. Zum Beispiel kann ein Teil des Laserstrahls mit einem teildurchlässigen Spiegel 24 ausgeblendet und auf ein Testtarget gerichtet werden und die optische Wirkung des Laserstrahls auf diesem Testtarget ist quasi ein Maß auch für die Positionierung des anderen Teils des Laserstrahls auf der behandelten Cornea. Ein ungewolltes Verschieben der Ablationszone kann dabei berücksichtigt werden und entsprechend kann dann der Laserstrahl durch eine abgewandelte Steuerung der optischen Elemente 16 mittels des Rechners 20 nachgeführt werden, sodass die Ablationszone jeweils wie vorgesehen zentriert ist.

Zu den Eigenschaften der Laserstrahlung im weiteren Sinne gehören auch Merkmale, die den Strahlengang der Laserstrahlung betreffen, wie zum Beispiel das im Strahlengang befindliche Gas. Ist eine Stickstoffspülung für den Strahlengang der Laserstrahlung vorgesehen, dann kann dessen Konzentration gemessen werden und diese kann korreliert werden zur gemessenen Laserleistung. Ändert sich die Laserleistung, kann entweder die Stickstoffkonzentration geändert werden oder die Laserleistung selbst.

Weiterhin kann der Sensor 18 eine interne Diagnose bezüglich des Behandlungslaserstrahles 12 durchführen, zum Beispiel die Qualität des Laserstrahles in an sich bekannter Weise messen und eine Nachregelung durchführen, insbesondere hinsichtlich der oben genannten Parameter der Laserstrahlung. Wird innerhalb vorgegebener Toleranzen ein Mindestwert oder Höchstwert bezüglich eines Strahlungsparameters unterschritten bzw. überschritten, kann das Programm für diesen Fall entweder eine Warnung vorsehen oder ein Abschalten des Lasers, wie oben beschrieben.

Die vorstehend beschriebenen einzelnen Sensoren und ihr Auswertungen können in beliebiger Kombination und Auswahl von Steuerparametern eingesetzt werden.

Eine andere Weiterbildung der oben beschriebenen Ausführungsbeispiele sieht ein Mikrodisplay vor, das in das üblicherweise bei der beschriebenen Vorrichtung verwendete Operationsmikroskop eingeblendet ist. Neben den oben beschriebenen Parametern und Eigenschaften der Strahlung bzw. der Cornea können darüber hinaus weitere Daten eingeblendet sein, wie zum Beispiel Patientendaten, Angaben über Behandlungszonen, Daten über die Astigmatismusachse, eine maximale Behandlungszone etc.

## Patentansprüche

1. Vorrichtung zur ophthalmologischen Laserchirurgie mit einem Läser (14) zum Emittieren eines refraktiv wirkenden Laserstrahls (12), optischen Elementen (16) zum Formen und Richten des Laserstrahls auf ein Auge, einem Rechner (20) zum Steuern des Lasers und/oder der optischen Elemente (16) und zumindest einem ersten Sensor (18), der eingerichtet ist, eine Eigenschaft des Laserstrahls während der Chirurgie zu ermitteln und ein dieser Eigenschaft entsprechendes Signal an den Rechner (20) zu geben, **dadurch gekennzeichnet, dass** der Rechner programmiert ist, um den Laser (14) und/oder die optischen Elemente (16) in Abhängigkeit von dem Signal des ersten Sensors unter Anpassung des anschließenden Behandlungsverlaufs derart zu steuern dass bei Erhöhung der Energie der Laserpulse während der Behandlung, die, auschliessende Zahl der Laserpulse entsprechend verringert wird wobei die Eigenschaft des Laserstrahls dessen Energie und/oder Qualität ist.

2. Vorrichtung nach Anspruch 1, **dadurch gekennzeichnet, dass** der erste Sensor eine Kamera ist.

3. Vorrichtung nach Anspruch-2, **dadurch gekennzeichnet, dass** der Rechner (20) auch dazu eingerichtet ist, durch Bildverarbeitung eine Ist-Position des Laserstrahls auf der Kornea des Auges mit einer Soll-Position zu vergleichen.

4. Vorrichtung nach Anspruch 2, **dadurch gekennzeichnet, dass** der Rechner (20) dazu eingerichtet ist, die Anzahl der am selben Ort auf die Kornea auftreffenden Laserpulse mit einer vorgegebenen Anzahl zu vergleichen.

5. Vorrichtung nach einem der vorhergehenden Ansprüche, **gekennzeichnet durch** einen zweiten Sensor, der eingerichtet ist, eine Eigenschaft des Auges während der Chirurgie zu ermitteln und ein dieser Eigenschaft entsprechendes Signal an den Rechner (20) zu geben, wobei der Rechner programmiert ist, um den Laser (14) und/oder die optischen Elemente (16) in Abhängigkeit von dem Signal des zweiten Sensors unter Abänderung des laufenden chirurgischen Eingriffs gegenüber einem ursprünglichen Bearbeitungsprogramm zu steuern.

6. Vorrichtung nach Anspruch 5, **dadurch gekennzeichnet, dass** der zweite Sensor eine oder mehrere der folgenden Einrichtungen umfasst: ein Pachymeter, ein Spektrometer, ein Aberrometer, eine Einrichtung zur Messung einer Ablationstiefe.

## Claims

1. Apparatus for ophthalmological laser surgery, comprising a laser (14) for emitting a refractively operative laser beam (12), optical elements (16) for forming and directing the laser beam onto an eye, a computer (20) for controlling the laser and/or the optical elements (16), and at least a first sensor (18) adapted to sense a property of the laser beam during surgery and to input a signal to the computer (20) corresponding to said property, **characterised in that** the computer is programmed to control the laser (14) and/or the optical elements (16) in dependency from the signal of the first sensor by adapting the subsequent surgery such that upon increase of the energy of the laser pulses during the surgery the subsequent number of the laser pulses is reduced correspondingly, wherein the property of the laser beam is its energy.

2. Apparatus in accordance with claim 1, **characterised in that** the first sensor is a camera.

3. Apparatus according to claim 2, **characterised in that** the computer (20) is also adapted to compare an actual position of the laser beam at the cornea of the eye with a wanted position of the beam.

4. Apparatus in accordance with claim 2, **characterised in that** the computer (20) is adapted to compare the number of laser pulses impinging at a certain position at the cornea with a pre-given number.

5. Apparatus in accordance with one of the preceding claims, **characterised by** a second sensor adapted to determine, during surgery, a property of the eye and to transfer a signal to the computer (20) corresponding to that property, wherein the computer is programmed to control the laser (14) and/or the optical elements (16) in response to the signal of the second sensor by amending the current surgical operation as compared to the original surgical programme.

6. Apparatus in accordance with claim 5, **characterised in that** the second sensor comprises one or more of the following means: a pachymeter, a spectrometer, an aberrometer, or means for measuring ablation depths.

## Revendications

1. Dispositif pour la chirurgie ophtalmologique par laser, comprenant un laser (14) pour émettre un rayon laser (12) à effet réfractif, des éléments optiques (16) pour former et diriger le rayon laser sur un oeil, un calculateur (20) pour commander le laser et/ou les éléments optiques (16) et au moins un premier capteur (18) qui est équipé pour déterminer une propriété du rayon laser pendant l'intervention chirurgicale et envoyer au calculateur (20) un signal correspondant à cette propriété, **caractérisé en ce que** le calculateur est programmé pour commander le laser (14) et/ou les éléments optiques (16) en fonction du signal du premier capteur, lors de l'adaptation du déroulement du traitement consécutif, de telle manière que le nombre de pulsations laser suivant est réduit en conséquence lorsqu'augmente l'énergie des pulsations laser, l'énergie et/ou la qualité du rayon laser représentant la propriété du rayon laser.

2. Dispositif selon la revendication 1, **caractérisé en ce que** le premier capteur est une caméra.

3. Dispositif selon la revendication 2, **caractérisé en ce que** le calculateur (20) est également équipé pour procéder à un traitement d'images consistant à comparer une position théorique et une position réelle du rayon laser sur la cornée de l'oeil.

4. Dispositif selon la revendication 2, **caractérisé en ce que** le calculateur (20) est équipé pour comparer un nombre prédéfini et le nombre des pulsations laser atteignant le même endroit sur la cornée.

5. Dispositif selon l'une des revendications précédentes, **caractérisé par** un deuxième capteur qui est équipé pour déterminer une propriété de l'oeil pendant l'intervention chirurgicale et envoyer au calculateur (20) un signal correspondant à cette propriété, le calculateur étant programmé pour commander le laser (14) et/ou les éléments optiques (16) en fonction du signal du deuxième capteur, lors de la modification de l'intervention chirurgicale en cours par rapport à un programme de traitement initial.

6. Dispositif selon la revendication 5, **caractérisé en ce que** le deuxième capteur comprend un ou plusieurs des équipements suivants : un pachymètre, un spectromètre, un aberromètre, un dispositif servant à mesurer une profondeur d'ablation.
